# EUROPEAN PATENT APPLICATION

(11) **EP 3 912 542 A1**
(43) Date of publication of application: **24.11.2021**
(21) Application number: 19910044.7
(22) Date of filing: 26.09.2019
(51) Int. Cl.: A61B 1/04, G02B 23/26, G03B 17/02, H04N 5/225

(54) **CAMERA MODULE, AND CABLE CONNECTION METHOD FOR CAMERA MODULE AND CAMERA**

(30) Priority: 16.01.2019 JP 2019005228
(71) Applicant: Panasonic i-PRO Sensing Solutions Co., Ltd., Fukuoka-shi, Fukuoka 812-8531 (JP)
(72) Inventor: KUBARA, Takashi, Fukuoka 812-8531 (JP); KAWAUCHI, Yosuke, Fukuoka 812-8531 (JP); HATASE, Yuichi, Fukuoka 812-8531 (JP)
(74) Representative: Eisenführ Speiser
(86) International application number: PCT/JP2019/038047
(87) International publication number: WO 2020/148945

(57) **Abstract**

This camera module comprises: an imaging element which is formed in a rectangular shape and to which is provided a plurality of pads on the rear, which is the reverse side from the image capture surface; a wire positioning/fixing body which fixes together a plurality of parallel wires extending in a direction approximately perpendicular to the rear surface, and has each of the conductors of the wires protrude from the opposing end surface which is parallel to the rear surface in accordance with to each of the pads; and electro-conductive materials which conduct electricity from the tip of the conductors to each of the plurality of pads.

## Description

### TECHNICAL FIELD

The present disclosure relates to a camera module, a camera, and a cable connection method for the camera module.

### BACKGROUND ART

Generally, in an electronic endoscope, an imaging element is fixed in a metal-made shield pipe in a state where a positional relationship (that is, focusing) with an objective optical system is accurately adjusted, and a signal cable is joined to terminals arranged on a back surface of the imaging element from a rear side (for example, see PTL 1). Core wires of the signal cable are joined to the terminals arranged on the back surface of the imaging element by soldering or the like. Further, a joint portion between the core wires and the terminals is reinforced by molding a periphery thereof with an adhesive in order to prevent the joint portion from being broken during work of assembling the imaging element in the shield pipe.

### CITATION LIST

### PATENT LITERATURE

[PTL 1] JP-A-2004-159970

### SUMMARY OF INVENTION

### TECHNICAL PROBLEM

However, for example, in a fairly thin electronic endoscope used for insertion into a fairly small-diameter duct or hole such as a blood vessel, a quadrangle in which one side of an imaging element is 1 mm or less may be used. In this case, in order to solder, by manual work, tip ends of the core wires of the signal cable to the terminals (that is, pads) arranged on the back surface of the imaging element, a considerably proficient skill is required and a large number of mounting man-hours are required. For example, microfabrication is required to expose a part of the core wires of the signal cable one by one. Therefore, in the related-art connection structure and connection method of the imaging element and the signal cable of the electronic endoscope, there is a problem that automation is difficult and a manufacturing cost is increased in a camera such as a fairly thin electronic endoscope assumed to be used as described above because human intervention of an operator who requires a considerably proficient skill is required.

The present disclosure has been made in view of the related-art situations described above, and an object thereof is to provide a camera module, a camera, and a cable connection method for the camera module that can implement mass production and reduce a manufacturing cost by enabling positioning between a pad and a conductor without requiring a proficient skill and easily enabling an assembly by automation.

### SOLUTION TO PROBLEM

The present disclosure provides a camera module including: an imaging element formed in a quadrangular shape and provided with a plurality of pads on a back surface opposite to an imaging surface; an electric wire positioning and fixing body in which a plurality of parallel electric wires that extend in a direction substantially perpendicular to the back surface are integrally fixed, and conductors of the electric wires protrude from a facing end surface parallel to the back surface according to the pads; and conductive materials configured to conductively connect tip ends of the conductors to the plurality of pads.

Further, the present disclosure provides a camera including: the camera module; and a lens disposed on an imaging surface side of the imaging element.

Further, the present disclosure provides a cable connection method for a camera module that connects a cable including a plurality of electric wires to a camera module including an imaging element, the cable connection method including: an electric wire collectively supplying step of supplying an electric wire positioning and fixing body in which the electric wires whose conductors are exposed at a tip end are collectively fixed; a conductive material applying step of applying unsolidified conductive materials to at least one of the conductors and a plurality of pads provided on a back surface of the imaging element; a conductor positioning step of positioning the tip ends of the conductors that protrude from the electric wire positioning and fixing body to the pads; and a conductive material fixing step of blowing high-temperature air having a temperature higher than a melting point of the conductive materials to the conductive materials to conductively connect the pads and the conductors via the melted conductive materials.

### ADVANTAGEOUS EFFECTS OF INVENTION

According to the present disclosure, it is possible to position a pad and a conductor without requiring a proficient skill and to easily perform an assembly by automation, so that mass production can be implemented and a manufacturing cost can be reduced.

### BRIEF DESCRIPTION OF DRAWINGS

[FIG. 1] FIG. 1 is a perspective view showing an appearance example of an endoscope system according to a first embodiment.
[FIG. 2] FIG. 2 is a perspective view showing an appearance example of a tip end side of a camera shown in FIG. 1.
[FIG. 3] FIG. 3 is a side view showing a camera module mounted on the camera shown in FIG. 2 together with a part of a cable.
[FIG. 4] FIG. 4 is a rear view showing a back surface of an imaging element shown in FIG. 3.
[FIG. 5] FIG. 5 is a front view and a side view showing the camera module in which a lens and the imaging element shown in FIG. 3 are omitted, together with the cable.
[FIG. 6] FIG. 6 is a step diagram showing an example of a procedure in a cable connection method for the camera module shown in FIG. 5.
[FIG. 7] FIG. 7 is a step diagram showing an example of a manufacturing procedure of an electric wire positioning and fixing body shown in FIG. 6.
[FIG. 8] FIG. 8 is a front view and a side view showing the camera module according to a first modification together with the cable.
[FIG. 9] FIG. 9 is a step diagram showing an example of a manufacturing procedure of an electric wire positioning and fixing body shown in FIG. 8.
[FIG. 10] FIG. 10 is a front view and a side view showing the camera module according to a second modification together with the cable.
[FIG. 11] FIG. 11 is a step diagram showing an example of a manufacturing procedure of an electric wire positioning and fixing body shown in FIG. 10.

### DESCRIPTION OF EMBODIMENTS

Hereinafter, embodiments in which a configuration and functions of a camera module, a camera, and a cable connection method for the camera module according to the present disclosure are specifically disclosed will be described in detail with reference to the drawings as appropriate. However, unnecessarily detailed description may be omitted. For example, detailed description of a well-known matter or repeated description of substantially the same configuration may be omitted. This is to avoid unnecessary redundancy in the following description and to facilitate understanding of those skilled in the art. It is to be understood that the accompanying drawings and the following description are provided to enable those skilled in the art to fully understand the present disclosure, and are not intended to limit the range of the claims.

### (First Embodiment)

First, an endoscope system 11 and a camera 13 (for example, an endoscope) that constitutes the endoscope system 11 according to a first embodiment will be described.

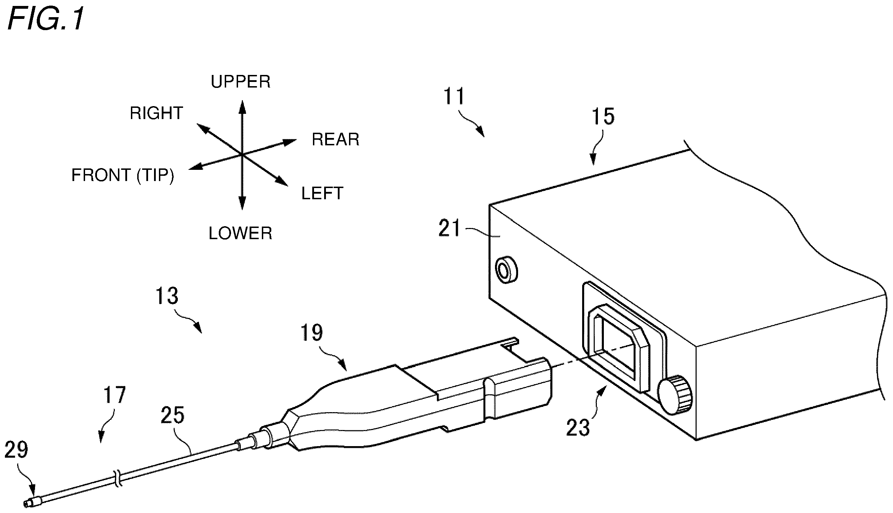

FIG. 1 is a perspective view showing an appearance example of the endoscope system 11 according to the first embodiment. In FIG. 1, an overall configuration of the endoscope system 11 including the camera 13 and a video processor 15 is shown in the perspective view. Directions used in the description of the present specification follow the description of the directions in FIG. 1. Here, "upper" and "lower" respectively correspond to an upper side and a lower side of the video processor 15 placed on a horizontal surface, and "front (tip)" and "rear" respectively correspond to a tip end side of an insertion portion 17 of the camera 13 and a base end side (in other words, a video processor side) of a plug portion 19.

As shown in FIG. 1, the endoscope system 11 includes, for example, the camera 13 (for example, the endoscope) that is a medical flexible scope, and the video processor 15 that performs a known image processing or the like on a still image or a moving image obtained by capturing an image of an inside of an observation target (for example, a blood vessel of a human body). The camera 13 includes the insertion portion 17 that extends substantially in a front-rear direction and is inserted into the inside of the observation target, and the plug portion 19 to which a rear portion of the insertion portion 17 is connected.

The video processor 15 includes a socket portion 23 that opens in a front wall 21. A rear portion of the plug portion 19 of the camera 13 is inserted into the socket portion 23. Accordingly, the camera 13 can transmit and receive electric power and various signals (a video signal, a control signal, and the like) to and from the video processor 15.

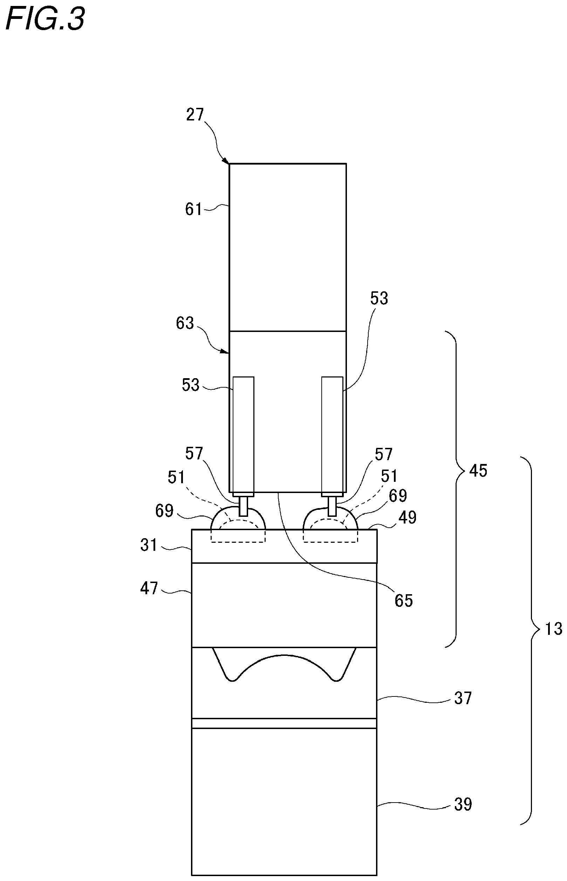

The above-described electric power and various control signals are guided from the plug portion 19 to a flexible portion 25 via a cable 27 (see FIG. 3) inserted into an inside of the flexible portion 25. Data of a captured image output from an imaging element 31 (see FIG. 3) provided at a tip end portion 29 is transmitted from the plug portion 19 to the video processor 15 via the cable 27. The video processor 15 performs a known image processing such as color correction and gradation correction on the data of the captured image transmitted from the plug portion 19, and outputs the data of the captured image after the image processing to a display apparatus (not shown). The display apparatus is, for example, a monitor apparatus including a display device such as a liquid crystal display panel, and displays data of a captured image of a subject captured by the camera 13 (for example, a captured image showing a state in the blood vessel of the human body that is the subject).

The insertion portion 17 includes the flexible portion 25 that has flexibility and has a rear end connected to the plug portion 19, and the tip end portion 29 connected to a tip end of the flexible portion 25. The flexible portion 25 has an appropriate length corresponding to various methods such as an endoscopic examination and an endoscopic surgery. The flexible portion 25 is configured by, for example, covering an outer periphery of a metal thin plate wound in a spiral shape with a net and further covering the outer periphery with a coating, and is formed to have sufficient flexibility. The flexible portion 25 performs connection between the tip end portion 29 and the plug portion 19.

The camera 13 is formed to have a small diameter, so that insertion into a body cavity having a small diameter is enabled. The body cavity having the small diameter is not limited to the blood vessel of the human body, and includes, for example, a urinary duct, a pancreatic duct, a bile duct, and a bronchiole. That is, the camera 13 can be inserted into the blood vessel, the urinary duct, the pancreatic duct, the bile duct, the bronchiole, and the like of the human body. In other words, the camera 13 can be used for observing a lesion in the blood vessel. The camera 13 is effective in identifying an arteriosclerotic plaque. Further, the camera 13 can also be applied to observation by an endoscope during a cardiac catheter examination. Furthermore, the camera 13 is also effective in detecting a thrombus and an arteriosclerotic yellow plaque. In an arteriosclerotic lesion, a color tone (white, pale yellow, yellow) and a surface (smoothness, irregularity) are observed. In the thrombus, a color tone (red, white, dark red, yellow, brown, mixed color) is observed.

The camera 13 can be used for diagnosis and treatment of a renal pelvis/urinary duct cancer and essential renal bleeding. In this case, the camera 13 can be inserted into a bladder from a urethra and further advanced into the urinary duct to observe insides of the urinary duct and the renal pelvis.

The camera 13 can be inserted into a papilla of Vater that opens in a duodenum. Bile is produced from a liver and passes through the bile duct, pancreatic juice is produced from a pancreas and passes through a pancreatic duct, and the bile and the pancreatic juice are discharged from the papilla of Vater in the duodenum. The camera 13 is inserted from the papilla of Vater, which is an opening of the bile duct and the pancreatic duct, so that the bile duct or the pancreatic duct can be observed.

In addition, the camera 13 can be inserted into a bronchus. The camera 13 is inserted from an oral cavity or a nasal cavity of a specimen (that is, a surgical target person) in a supine position. The camera 13 passes through a pharynx and a larynx and is inserted into a trachea while visually recognizing a vocal cord. The bronchus becomes thinner each time it branches. For example, according to the camera 13 having a maximum outer diameter of less than 2 mm, it is possible to check a lumen up to a subsegmental bronchus.

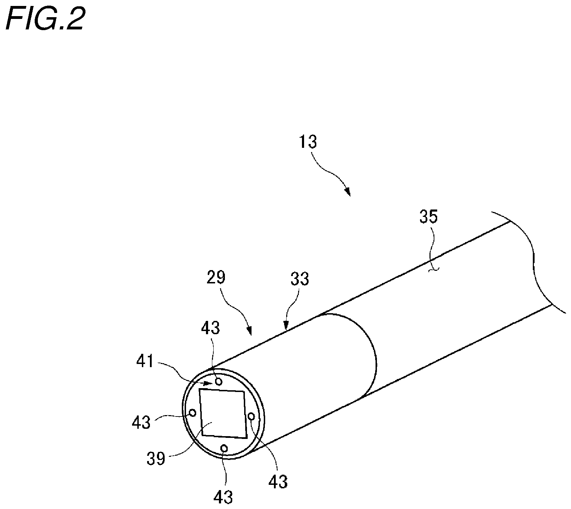

FIG. 2 is a perspective view showing an appearance example of a tip end side of the camera 13 shown in FIG. 1. The camera 13 includes a molded portion 33 at the tip end portion 29. A tubular sheath 35 that is formed with the same outer diameter as that of the tip end portion 29 and covers at least a part of the molded portion 33 is connected to the molded portion 33. The molded portion 33 is made of a mold resin, and is formed into a columnar shape by embedding a lens 37 and the imaging element 31, which will be described later. An inner diameter side of the sheath 35 is fixed to an outer periphery of a small-diameter extension portion (not shown) that extends from a rear end of the molded portion 33 by an adhesive or the like. The cable 27, which will be described later, is inserted into an inside of the sheath 35.

A quadrangular objective cover glass 39 is provided at a central portion of a tip end surface of the tip end portion 29. On the tip end surface, light-emitting end surfaces of four optical fibers 43 that constitute a light guide 41 are arranged outside sides of the objective cover glass 39.

FIG. 3 is a side view showing a camera module 45 mounted on the camera 13 shown in FIG. 2 together with a part of the cable 27. The camera 13 includes the lens 37 and the camera module 45. The lens 37 is provided behind the objective cover glass 39 disposed closest to an objective side of the tip end portion 29, and is configured with a single lens. The imaging element 31 is provided behind the lens 37 with an element cover glass 47 for protecting an imaging surface of the imaging element sandwiched therebetween. That is, the camera 13 is configured by disposing the lens 37 on an imaging surface side of the imaging element 31 provided in the camera module 45. In the camera 13, the objective cover glass 39, the lens 37, the camera module 45, and a part of the cable 27 are embedded in the molded portion 33 that forms the tip end.

The imaging element 31 provided in the camera module 45 is formed in a quadrangular shape (for example, a square shape) having one side of 1 mm or less (for example, about 0.5 mm to 1 mm). In the imaging element 31, a plurality of pads 51 are provided on a back surface 49 opposite to the imaging surface.

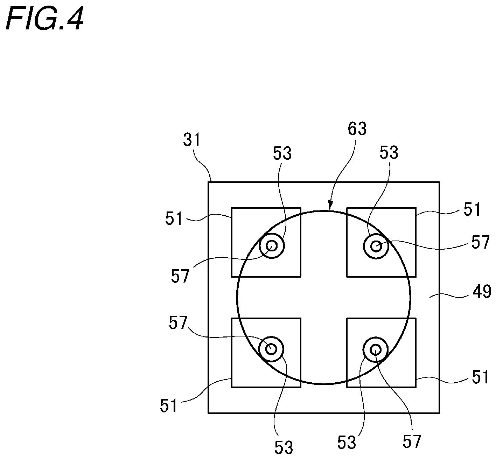

FIG. 4 is a rear view showing the back surface 49 of the imaging element 31 shown in FIG. 3. The plurality of pads 51 (four pads in the illustrated example of FIG. 4) are arranged, for example, one at each of four corners of the substantially square back surface 49 of the imaging element 31. The pads 51 are used for circuit connection of, for example, a power supply, an image signal, GND (ground), CLK (for example, input of a clock and a command from the video processor 15), and the like. The number and arrangement of the pads 51 are not limited thereto.

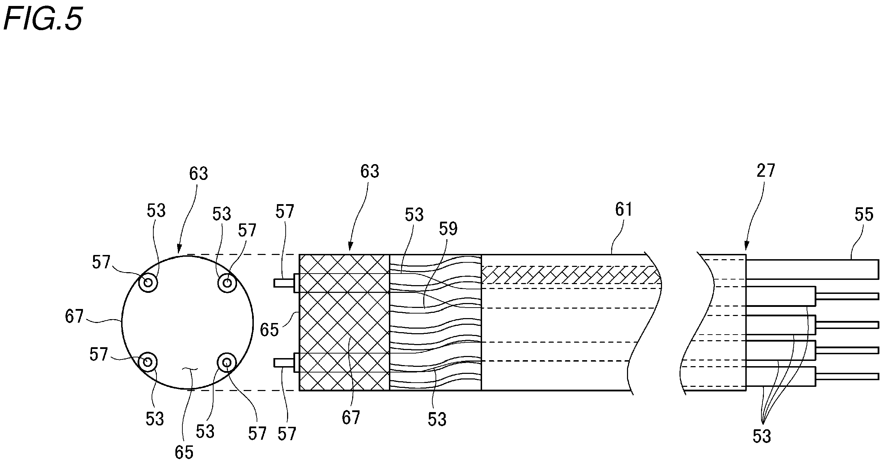

The cable 27 is conductively connected to the pads 51 provided on the back surface 49 of the imaging element 31. The cable 27 includes a plurality of electric wires 53 and a shield wire 55 (see FIG. 5). In each of the electric wires 53, an outer periphery of a linear conductor 57 is covered by an inner coating. The shield wire 55 is wired as it is without an element wire made of aluminum, copper, or the like being covered by a coating. Outer peripheries of the shield wire 55 and the plurality of electric wires 53 covered by the inner coating and insulated from each other are collectively covered by a shield layer 59.

FIG. 5 is a front view and a side view showing the camera module 45 in which the lens 37 and the imaging element 31 shown in FIG. 3 are omitted, together with the cable 27. The shield layer 59 prevents electrostatic breakdown of an electronic component such as the imaging element 31 due to application of static electricity from an outside to the electric wires 53. For the shield layer 59, for example, a metal foil such as aluminum or a braided shield in which a small-diameter lead wire or the like is knitted in a mesh shape is used. Further, the shield layer 59 may be provided with two layers of the metal foil and the braided shield. An outer periphery of the shield layer 59 that covers the plurality of electric wires 53 and the shield wire 55 is further covered by a tubular cable sheath 61 made of a soft insulating resin. That is, the cable 27 is configured with a three-layer structure including, from a center side, the plurality of electric wires 53 and the shield wire 55, the shield layer 59 that covers the plurality of electric wires 53 and the shield wire 55, and the cable sheath 61 that covers the outer periphery of the shield layer 59.

Abase end of the cable 27 on a rear end side is introduced into the plug portion 19 described above. In the cable 27 introduced into the plug portion 19, the conductors 57 of the electric wires 53 are soldered to a predetermined circuit terminal of a circuit board (not shown) housed in the plug portion 19. Further, the shield wire 55 is soldered to a GND terminal of the circuit board. Accordingly, the data of the captured image output from the imaging element 31 is transmitted from the plug portion 19 to the video processor 15 via the cable 27.

Incidentally, at a tip end of the cable 27 used for the transmission of the data of the captured image, the electric wires 53 are connected to the pads 51 provided on the back surface 49 of the imaging element 31. The camera module 45 includes an electric wire positioning and fixing body 63 for connecting the plurality of electric wires 53 to the pads 51 of the imaging element 31. That is, the camera module 45 is configured with the imaging element 31 and the electric wire positioning and fixing body 63. The plurality of electric wires 53 are guided by the electric wire positioning and fixing body 63 and connected to the imaging element 31.

In the electric wire positioning and fixing body 63, the plurality of parallel electric wires 53 that extend in a direction substantially perpendicular to the back surface 49 are integrally fixed. The electric wire positioning and fixing body 63 causes the conductors 57 of the electric wires 53 to protrude from a facing end surface 65 parallel to the back surface 49 according to the pads 51.

In the first embodiment, the electric wire positioning and fixing body 63 is an insulating adhesive material 67 solidified by collectively covering the plurality of conductors 57. For the adhesive material 67, for example, an epoxy-based one (including a one-component one and a two-component one) or an acrylic one can be used.

The electric wire positioning and fixing body 63 is formed in a cylindrical shape having a circular shape in an orthogonal direction of the electric wires 53, and the four electric wires 53 are in contact with an inner side of the circular shape.

As shown in FIG. 4, the electric wire positioning and fixing body 63 formed in the cylindrical shape is formed to have a diameter smaller than a length of a shortest side of the imaging element 31.

In the plurality of electric wires 53 collected by the electric wire positioning and fixing body 63, the conductors 57 that protrude from the facing end surface 65 are conductively connected to the plurality of pads 51 by conductive materials 69. A low-melting-point conductive material is preferably used for the conductive material 69. The low-melting-point conductive material can be, for example, cream solder obtained by kneading solder powder into a paste-like flux. The cream solder is used, so that a fine pattern can be transferred to the pads 51 and the conductors 57 by a printing apparatus.

Next, a cable connection method for the camera module 45 according to the first embodiment will be described.

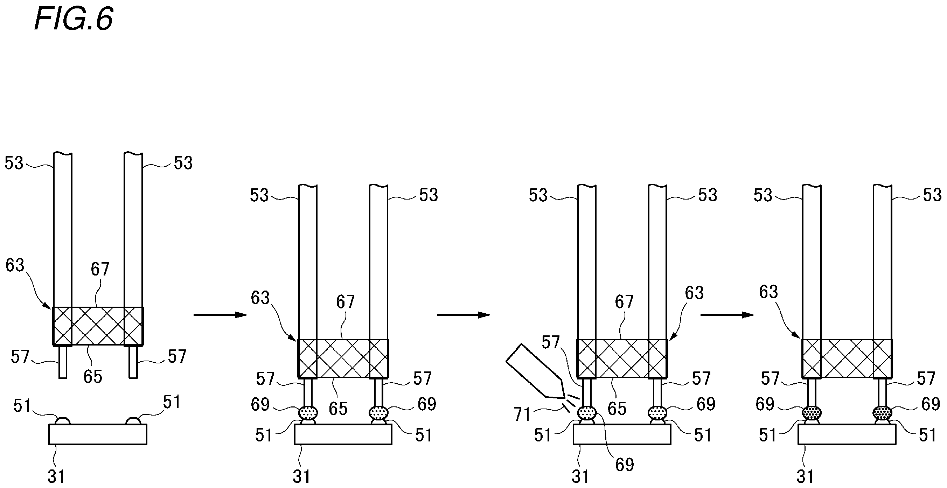

FIG. 6 is a step diagram showing an example of a procedure in the cable connection method for the camera module 45 shown in FIG. 5. The cable connection method for the camera module includes an electric wire collectively supplying step, a conductive material applying step, a conductor positioning step, and a conductive material fixing step as main steps.

The imaging element 31 is placed on a turn table (not shown) or the like whose upper surface is a horizontal surface such that the back surface 49 is on an upper side. In the electric wire collectively supplying step, with respect to the imaging element 31, the electric wire positioning and fixing body 63 in which the electric wires 53 whose conductors 57 are exposed at the tip end are collectively fixed, is supplied from above in FIG. 6.

In the conductive material applying step, the unsolidified conductive materials 69 are applied to at least one of the conductors 57 and the plurality of pads 51 provided on the back surface 49 of the imaging element 31.

In the conductor positioning step, tip ends of the conductors 57 that protrude from the electric wire positioning and fixing body 63 are positioned to the pads 51 with the electric wire positioning and fixing body 63 standing perpendicularly.

In the conductive material fixing step, high-temperature air 71 having a temperature higher than a melting point of the conductive materials 69 is blown to the conductive materials 69, and the pads 51 and the conductors 57 are conductively connected via the melted conductive materials 69.

Accordingly, according to the cable connection method for the camera module 45 in the first embodiment, the conductors 57 of the plurality of electric wires 53 can be conductively connected to the pads 51, and connection of the cable 27 to the camera module 45 is completed.

In the cable connection method for the camera module 45, in the conductive material fixing step, the pads 51 and the conductors 57 may be conductively connected via the conductive materials 69 irradiated with laser light having a temperature higher than the melting point of the conductive materials 69 and melted.

In the cable connection method for the camera module 45, in the conductive material fixing step, the pads 51 and the conductors 57 may be conductively connected via the conductive materials 69 heated by a reflow furnace and melted.

Here, the cable connection method for the camera module 45 can include a procedure of fixing the plurality of electric wires 53 collectively by the electric wire positioning and fixing body 63 in the above-described procedure. In this case, an electric wire collectively fixing step is added before the above-described electric wire collectively supplying step. In the procedure of the cable connection method for the camera module described above, an example in which the electric wire collectively fixing step is performed in a separate step is shown. That is, the electric wire positioning and fixing body 63 manufactured in a separate step is supplied in the electric wire collectively supplying step.

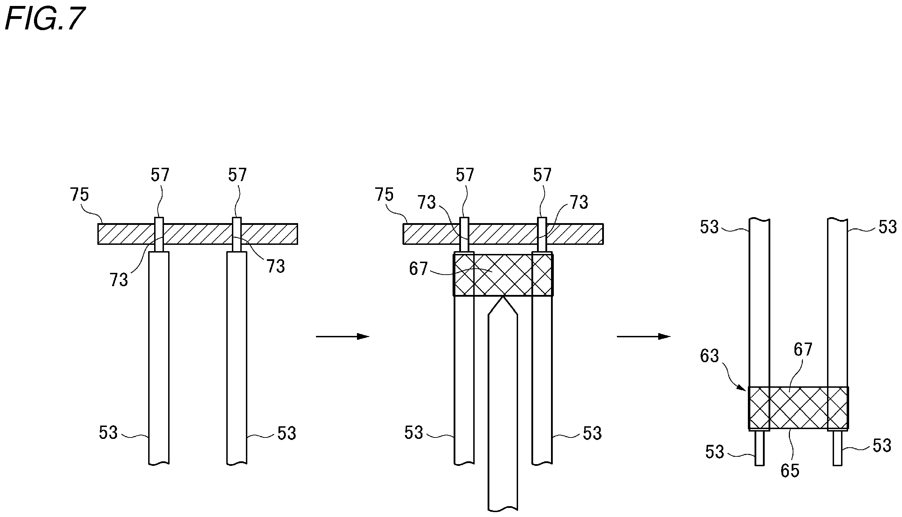

FIG. 7 is a step diagram showing an example of a manufacturing procedure of the electric wire positioning and fixing body 63 shown in FIG. 6. In the electric wire collectively fixing step of manufacturing the electric wire positioning and fixing body 63, first, the electric wires 53 are inserted into insertion holes 73 of a jig 75 in which a plurality of insertion holes 73 into which the electric wires 53 are inserted are bored such that the electric wires 53 face the pads 51. Next, the electric wires before being inserted into the jig 75 are collectively covered by the insulating adhesive material 67. At this time, the jig 75 is disposed on an upper side and the adhesive material 67 is injected from a lower side, so that it is possible to easily prevent contact between the adhesive material 67 and the jig 75. After the adhesive material 67 is solidified, the jig 75 is removed from the electric wires 53, and manufacturing of the electric wire positioning and fixing body 63 is completed. As described above, the electric wire collectively fixing step can be incorporated before the electric wire collectively supplying step in the cable connection method for the camera module 45 described above.

Next, functions of configurations of the camera module 45 and the camera 13 described above will be described.

The camera module 45 according to the first embodiment includes the imaging element 31 formed in the quadrangular shape and provided with the plurality of pads 51 on the back surface 49 opposite to the imaging surface, the electric wire positioning and fixing body 63 in which the plurality of parallel electric wires 53 that extend in the direction substantially perpendicular to the back surface 49 are integrally fixed and the conductors 57 of the electric wires 53 protrude from the facing end surface 65 parallel to the back surface 49 according to the pads 51, and the conductive materials 69 that conductively connect the tip ends of the conductors 57 to the plurality of pads 51.

In the camera module 45 according to the first embodiment, the plurality of pads 51 are provided on the back surface 49 of the imaging element 31. The electric wire positioning and fixing body 63 is connected to the back surface 49 of the imaging element 31. In the electric wire positioning and fixing body 63, the plurality of parallel electric wires 53 that extend in a direction substantially perpendicular to the back surface 49 of the imaging element 31 are integrally fixed. That is, the independent electric wires 53 are collectively fixed. The electric wire positioning and fixing body 63 includes the facing end surface 65 parallel to the back surface 49.

Here, in the electric wire positioning and fixing body 63, the electric wires 53 are arranged so as to correspond to the pads 51 provided on the back surface 49. In the electric wires 53 arranged corresponding to the pads 51, the conductors 57 at the tip end, exposed by removing the coating, protrude from the facing end surface 65 of the electric wire positioning and fixing body 63 toward the back surface 49 of the imaging element 31. Accordingly, the electric wire positioning and fixing body 63 is positioned at a predetermined relative position with respect to the imaging element 31, so that positioning of the conductors 57 of the plurality of electric wires 53 to the plurality of pads 51 is completed at the same time. At this time, the conductive material 69 such as the cream solder is provided in advance on at least one of the conductor 57 and the pad 51 by application or the like. Therefore, after the positioning is completed, the conductor 57 and the pad 51 are conductively connected to each other by melting and solidifying the conductive material 69 by the high-temperature air 71.

In the related art, when one side of the imaging element 31 is about 1 mm, the imaging element 31 can be connected to the electric wires 53 by manual work using a microscope or the like. However, when one side is less than 0.5 mm due to further miniaturization of the imaging element 31, work of manually joining the electric wire 53 and the pad 51 becomes fairly inefficient or difficult.

Therefore, in the camera module 45, the conductors 57 of the plurality of electric wires 53 are held by the electric wire positioning and fixing body 63 according to positions of the pads 51. Therefore, when the electric wire positioning and fixing body 63 is positioned at a predetermined relative position with respect to the imaging element 31, all the conductors 57 can be collectively positioned with respect to the pads 51 without positioning one pad 51 and the conductor 57 of one electric wire 53 one by one. The predetermined relative position between the electric wire positioning and fixing body 63 and the imaging element 31 can be performed by, for example, an image processing of aligning the electric wire positioning and fixing body 63 with a marker provided on the back surface 49.

Therefore, in the camera module 45, it is possible to implement mass production and reduce a manufacturing cost by automating from positioning to connection of the plurality of electric wires 53 and the pads 51 without requiring a proficient skill.

In the camera module 45, the electric wire positioning and fixing body 63 is the insulating adhesive material 67 solidified by collectively covering the plurality of conductors 57.

In the camera module 45, the electric wire positioning and fixing body 63 collectively fixes the plurality of electric wires 53 by the adhesive material 67. Since the fixing structure can be formed only by the adhesive material 67 except for using the simple jig 75, the electric wire positioning and fixing body 63 can be manufactured inexpensively and in a small size (a short length).

In the camera module 45, the electric wire positioning and fixing body 63 is formed in the cylindrical shape having the circular shape in the orthogonal direction of the electric wires 53, and the four electric wires 53 are in contact with the inner side of the circular shape.

In the camera module 45, when the electric wire positioning and fixing body 63 has a cylindrical shape, the four electric wires 53 can be arranged to be most separated from each other. Accordingly, a maximum insulation distance among the electric wires can be secured. In other words, when a predetermined insulation distance needs to be secured, an arrangement structure of the electric wires 53 in which a cylindrical shape can be formed with a smallest outer diameter can be used. As a result, it is possible to obtain a structure advantageous in reducing a diameter of the camera 13.

In the camera module 45, a diameter of the electric wire positioning and fixing body 63 is smaller than the length of the shortest side of the imaging element 31.

In the camera module 45, the diameter of the electric wire positioning and fixing body 63 formed in the cylindrical shape is set to be smaller than the length of the shortest side of the imaging element 31. Therefore, the electric wire positioning and fixing body 63 can be disposed without protruding outward from the back surface 49 of the imaging element 31. Accordingly, the camera module 45 can have a structure in which the electric wire positioning and fixing body 63 does not interfere with another member (for example, the light guide 41) disposed outside sides of the quadrangular imaging element 31.

In the camera module 45, a length of a longest side of the imaging element 31 is 1 mm or less.

In the camera module 45, the imaging element 31 is formed in a quadrangular shape (for example, a square shape) in which the length of the longest side of the imaging element 31 is 1 mm or less (for example, about 0.5 mm to 1 mm). The camera 13 for a blood vessel on which the camera module 45 is mounted can be miniaturized to an outer diameter capable of being inserted into, for example, a coronary artery. In the camera module 45, for example, even when the cable 27 is connected to the imaging element 31 in which the length of the longest side is 0.5 mm, it is possible to automatically position the electric wires 53 on the plurality of pads 51 by using the electric wire positioning and fixing body 63.

The camera 13 according to the first embodiment includes the camera module 45 and the lens 37 disposed on the imaging surface side of the imaging element 31.

In the camera 13 according to the first embodiment, light from an image-capturing portion forms an image on the imaging surface of the imaging element 31 of the camera module 45 by the lens 37. The imaging element 31 converts the image-forming light into an electric signal, and outputs the electric signal as an image signal from the pads 51 on the back surface 49 to the cable 27 via the electric wires 53. In the camera 13, the cable 27 can be easily and highly accurately connected to the imaging element 31, which is a fine component, by using the electric wire positioning and fixing body 63. Accordingly, the camera 13 can be mass-produced by automation.

Therefore, according to the camera 13 in the first embodiment, the pads 51 and the conductors 57 can be positioned without requiring a proficient skill, and assembly by automation can be easily performed, so that mass production can be implemented and a manufacturing cost can be reduced.

The cable connection method for the camera module according to the first embodiment includes: the electric wire collectively supplying step of supplying the electric wire positioning and fixing body 63 in which the electric wires 53 whose conductors 57 are exposed at the tip end are collectively fixed; the conductive material applying step of applying the unsolidified conductive materials 69 to at least one of the conductors 57 and the plurality of pads 51 provided on the back surface 49 of the imaging element 31; the conductor positioning step of positioning the tip ends of the conductors 57 that protrude from the electric wire positioning and fixing body 63 to the pads 51; and the conductive material fixing step of blowing high-temperature air 71 having the temperature higher than the melting point of the conductive materials 69 to the conductive materials 69 to conductively connect the pads 51 and the conductors 57 via the melted conductive materials 69.

In the cable connection method for the camera module according to the first embodiment, when connecting the cable 27 to the imaging element 31 that is a fine component, the electric wire positioning and fixing body 63 in which the plurality of electric wires 53 are collectively fixed is supplied.

In the electric wire positioning and fixing body 63, the plurality of parallel electric wires 53 that extend in a direction substantially perpendicular to the back surface 49 of the imaging element 31 are integrally fixed. That is, the independent electric wires 53 are collectively fixed. The electric wire positioning and fixing body 63 includes the facing end surface 65 parallel to the back surface 49. Here, in the electric wire positioning and fixing body 63, the electric wires 53 are arranged so as to correspond to the pads 51 provided on the back surface 49. In the electric wires 53 arranged corresponding to the pads 51, the conductors 57 at the tip end, exposed by removing the coating, protrude from the facing end surface 65 of the electric wire positioning and fixing body 63 toward the back surface 49 of the imaging element 31.

The unsolidified conductive materials 69 are applied to at least one of the conductors 57 that protrude from the electric wire positioning and fixing body 63 and the plurality of pads 51 provided on the back surface 49 of the imaging element 31.

The electric wire positioning and fixing body 63 is positioned at a predetermined relative position with respect to the imaging element 31, so that positioning of the conductors 57 of the plurality of electric wires 53 to the plurality of pads 51 is completed at the same time.

After the positioning is completed, the conductors 57 and the pads 51 are conductively connected by melting and solidifying the conductive materials 69 by the high-temperature air 71.

In the cable connection method for the camera module, the conductors 57 of the plurality of electric wires 53 are held by the electric wire positioning and fixing body 63 according to the pads 51. Therefore, when the electric wire positioning and fixing body 63 is positioned at a predetermined relative position with respect to the imaging element 31, all the conductors 57 can be collectively positioned with respect to the pads 51 without positioning one pad 51 and the conductor 57 of one electric wire 53 one by one. The electric wire positioning and fixing body 63 can be mounted even on the small imaging element 31 such as a medical camera and can be miniaturized. Further, since a fixed position is predetermined, automation is possible and man-hours are not required.

Therefore, in the cable connection method for the camera module, it is possible to implement mass production and reduce a manufacturing cost by automating from positioning to connection of the plurality of electric wires 53 and the pads 51 without requiring a proficient skill.

In the cable connection method for the camera module, the electric wire positioning and fixing body 63 is formed by inserting the electric wires 53 into the insertion holes 73 of the jig 75 in which the plurality of insertion holes 73 into which the electric wires 53 are inserted are bored such that the electric wires 53 face the pads 51, collectively covering the electric wires before being inserted into the jig 75 with the insulating adhesive material 67, and removing the jig 75 from the electric wires 53 after the adhesive material 67 is solidified.

In the cable connection method for the camera module, the electric wire positioning and fixing body 63 in which the plurality of electric wires 53 are collectively fixed can be easily and inexpensively formed only by the adhesive material 67 except for using the simple jig 75. Further, a volume of the electric wire positioning and fixing body 63 can be adjusted by increasing or decreasing a supply amount of the adhesive material 67. Accordingly, the small-sized electric wire positioning and fixing body 63 having a predetermined electric wire fixing strength can be easily formed. In the camera module 45, since the electric wire positioning and fixing body 63 can be formed in a small size, a curvature radius of the mounted camera 13 can be reduced.

Next, a first modification of the above-described first embodiment will be described.

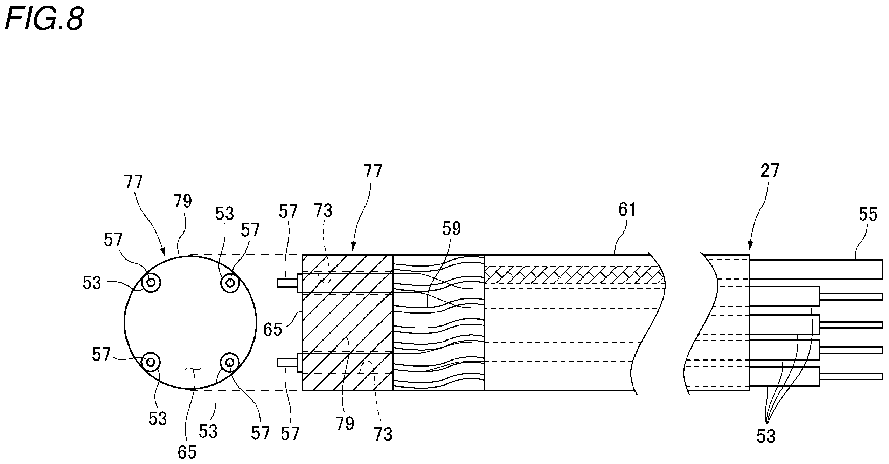

FIG. 8 is a front view and a side view showing the camera module 45 according to the first modification together with the cable 27. In the camera module 45 according to the first modification, an electric wire positioning and fixing body 77 is a columnar insulation member 79 made of an insulating resin material or the like including the plurality of insertion holes 73 through which the plurality of electric wires 53 are inserted.

In the camera module 45, the electric wire positioning and fixing body 77 is formed of the columnar insulation member 79. The columnar insulation member 79 includes the plurality of insertion holes 73 through which the electric wires 53 are inserted. The insertion holes 73 are bored at predetermined positions such that the conductors 57 of the electric wires 53 face the pads 51. Each electric wire 53 is fixed to the insertion hole 73 via the adhesive material 67. The columnar insulation member 79 can be formed in advance to have an outer shape of, for example, a highly accurate cylinder. Therefore, compared with a mold structure using the adhesive material 67, a shape of the electric wire positioning and fixing body 77 can be stabilized and manufactured in a small size (a short length).

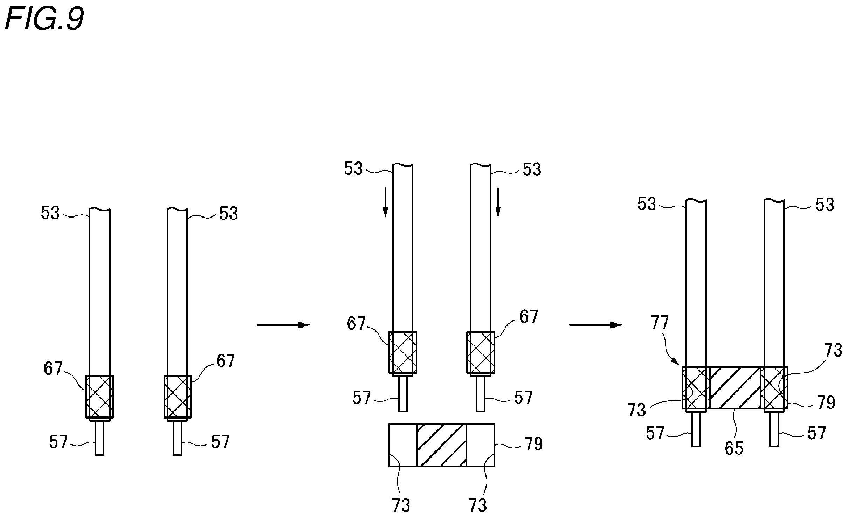

FIG. 9 is a step diagram showing an example of a manufacturing procedure of the electric wire positioning and fixing body 77 shown in FIG. 8. In the electric wire positioning and fixing body 77, the electric wires 53 are inserted into the insertion holes 73 of the insulation member 79 in which the plurality of insertion holes 73 into which the electric wires 53 are inserted are bored such that the electric wires 53 face the pads 51. The adhesive material 67 is applied to a coated tip end of the electric wire 53 in advance. The electric wires 53 and the insulation member 79 are fixed by the adhesive materials 67 to complete manufacturing of the electric wire positioning and fixing body 77. The electric wire collectively fixing step can be incorporated before the electric wire collectively supplying step in the cable connection method for the camera module described above.

In the cable connection method for the camera module, the columnar insulation member 79 can be formed in advance to have the outer shape of, for example, the highly accurate cylinder. Therefore, compared with the mold structure using the adhesive material 67, the shape of the electric wire positioning and fixing body 77 can be stabilized. Further, since time required for curing the adhesive material 67 can be shortened as compared with the mold structure, productivity can be improved.

Next, a second modification of the above-described first embodiment will be described.

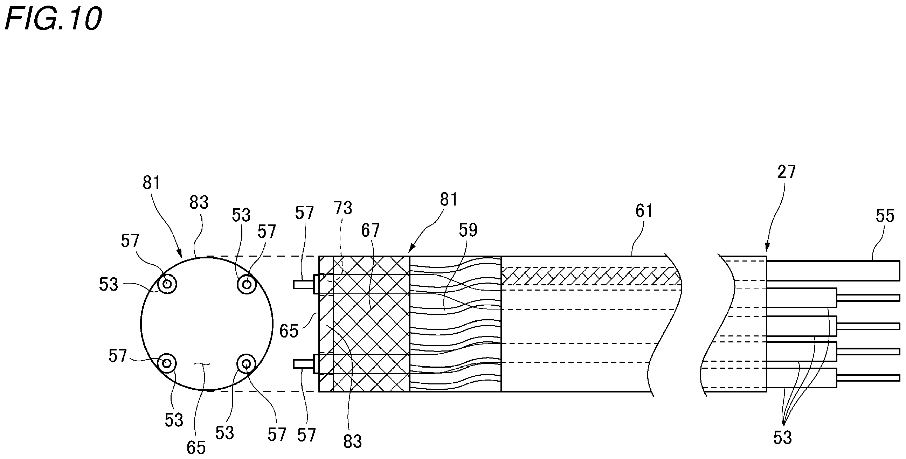

FIG. 10 is a front view and a side view showing the camera module 45 according to the second modification together with the cable 27. In the camera module 45 according to the second modification, an electric wire positioning and fixing body 81 includes, at a tip end thereof, an electric wire positioning plate 83 including the plurality of insertion holes 73 into which the plurality of electric wires 53 are inserted. In the electric wire positioning and fixing body 81, a side opposite to a penetration side of the electric wires 53 that penetrate through the insertion holes 73 is integrally fixed to the electric wire positioning plate 83 by the insulating adhesive material 67. As a material of the electric wire positioning plate 83, an insulating material such as polyimide or glass-epoxy resin having a heat resistance of 200°C or higher is used.

In the camera module 45, the electric wire positioning and fixing body 81 includes the plurality of insertion holes 73 into which the electric wires 53 are inserted in the electric wire positioning plate 83. The insertion holes 73 are bored at predetermined positions such that the conductors 57 of the electric wires 53 face the pads 51. That is, the electric wire positioning plate 83 serves as a jig that positions the conductors 57 to the pads 51. The electric wires 53 are integrally fixed to the electric wire positioning plate 83 by the adhesive material 67 in a state where the electric wires 53 are inserted into the insertion holes 73 of the electric wire positioning plate 83.

Therefore, in the electric wire positioning and fixing body 81 according to the second modification, since the facing end surface 65 facing the back surface 49 of the imaging element 31 is the electric wire positioning plate 83, the facing end surface 65 can be formed with a parallelism as high as the back surface 49 as compared with a mold structure using the adhesive material 67. Further, since the electric wire positioning plate 83 can be made thin, the electric wires 53 can be easily inserted into the insertion holes 73 as compared with a columnar insulating resin material. Further, as compared with a mold structure in which the entire electric wire positioning and fixing body 81 is formed of the adhesive material 67, an amount of the adhesive material 67 can be reduced because a strength is improved by using the electric wire positioning plate 83. Accordingly, the electric wire positioning and fixing body 81 can be manufactured in a small size (a short length) as compared with the mold structure using the adhesive material 67 and the structure using the columnar insulating resin material.

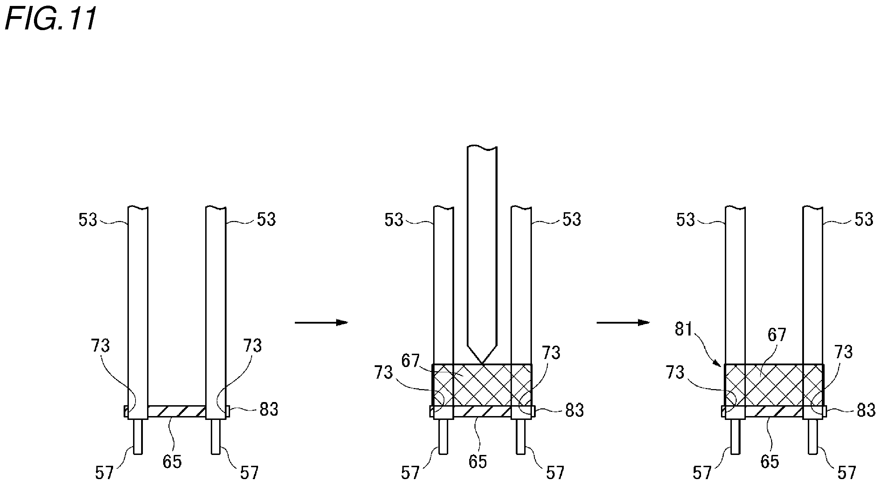

FIG. 11 is a step diagram showing an example of a manufacturing procedure of the electric wire positioning and fixing body 81 shown in FIG. 10.

In the electric wire positioning and fixing body 81, the electric wires 53 are inserted into the insertion holes 73 of the electric wire positioning plate 83 in which the plurality of insertion holes 73 into which the electric wires 53 are inserted are bored such that the electric wires 53 face the pads 51. The electric wire positioning and fixing body 81 adheres to the electric wire positioning plate 83 while collectively covering the electric wires before being inserted into the electric wire positioning plate 83 with the insulating adhesive material 67, so that manufacturing is completed. The electric wire collectively fixing step can be incorporated before the electric wire collectively supplying step in the cable connection method for the camera module described above.

In the cable connection method for the camera module, the electric wire positioning plate 83 serves as a jig that positions the conductors 57 to the pads 51. The electric wires 53 are integrally fixed to the electric wire positioning plate 83 by the adhesive material 67 in a state where the electric wires 53 are inserted into the insertion holes 73 of the electric wire positioning plate 83. In the electric wire positioning and fixing body 81, since the facing end surface 65 facing the back surface 49 of the imaging element 31 is the electric wire positioning plate 83, the facing end surface 65 can be formed with the parallelism as high as the back surface 49. Further, since the electric wire positioning plate 83 is used for the facing end surface 65, the adhesive material 67 can be injected while facing down, and a step of vertically inverting the electric wire positioning and fixing body 81 as shown in FIG. 7 can be omitted. Furthermore, as compared with the mold structure in which the entire electric wire positioning and fixing body 81 is formed of the adhesive material 67, the amount of the adhesive material 67 can be reduced because the strength is improved by using the electric wire positioning plate 83. Accordingly, compared with the mold structure using the adhesive material 67, the electric wire positioning and fixing body 81 can be manufactured in a small size (a short length).

Although the embodiment has been described above with reference to the accompanying drawings, the present disclosure is not limited to such examples. It will be apparent to those skilled in the art that various changes, modifications, substitutions, additions, deletions, and equivalents can be conceived within the scope of the claims, and it should be understood that such changes and the like also belong to the technical scope of the present disclosure. Further, components in the above-described embodiment may be optionally combined within a range not departing from the spirit of the invention.

The present application is based on a Japanese patent application (Japanese Patent Application No. 2019-005228) filed on January 16, 2019, the contents of which are incorporated by reference in the present application.

### INDUSTRIAL APPLICABILITY

The present disclosure is useful as a camera module, a camera, and a cable connection method for the camera module that can implement mass production and reduce a manufacturing cost by enabling positioning between a pad and a conductor without requiring a proficient skill and easily enabling an assembly by automation.

### REFERENCE SIGNS LIST

- 13: camera
- 27: cable
- 31: imaging element
- 37: lens
- 45: camera module
- 49: back surface
- 51: pad
- 53: electric wire
- 57: conductor
- 63: electric wire positioning and fixing body
- 65: facing end surface
- 67: adhesive material
- 69: conductive material
- 71: high-temperature air
- 73: insertion hole
- 75: jig
- 77: electric wire positioning and fixing body
- 79: insulation member
- 81: electric wire positioning and fixing body
- 83: electric wire positioning plate

## Claims

1. A camera module comprising:
an imaging element formed in a quadrangular shape and provided with a plurality of pads on a back surface opposite to an imaging surface;
an electric wire positioning and fixing body in which a plurality of parallel electric wires that extend in a direction substantially perpendicular to the back surface are integrally fixed, and conductors of the electric wires protrude from a facing end surface parallel to the back surface according to the pads; and
conductive materials configured to conductively connect tip ends of the conductors to the plurality of pads.

2. The camera module according to claim 1,
wherein the electric wire positioning and fixing body is an insulating adhesive material solidified by collectively covering the plurality of conductors.

3. The camera module according to claim 1,
wherein the electric wire positioning and fixing body is a columnar insulation member including a plurality of insertion holes into which the plurality of electric wires are inserted.

4. The camera module according to claim 1,
wherein the electric wire positioning and fixing body includes, at a tip end, an electric wire positioning plate including a plurality of insertion holes into which the plurality of electric wires are inserted, and a side opposite to a penetration side of the electric wires that penetrate through the insertion holes is integrally fixed to the electric wire positioning plate by an insulating adhesive material.

5. The camera module according to any one of claims 1 to 4,
wherein the electric wire positioning and fixing body is formed in a cylindrical shape having a circular shape in an orthogonal direction of the electric wires, and
wherein the four electric wires are in contact with an inner side of the circular shape.

6. The camera module according to claim 5,
wherein a diameter of the electric wire positioning and fixing body is smaller than a length of a shortest side of the imaging element.

7. The camera module according to any one of claims 1 to 6,
wherein a length of a longest side of the imaging element is 1 mm or less.

8. A camera comprising:
the camera module according to any one of claims 1 to 7; and
a lens disposed on an imaging surface side of the imaging element.

9. A cable connection method for a camera module that connects a cable including a plurality of electric wires to a camera module including an imaging element, the cable connection method comprising:
an electric wire collectively supplying step of supplying an electric wire positioning and fixing body in which the electric wires whose conductors are exposed at a tip end are collectively fixed;
a conductive material applying step of applying unsolidified conductive materials to at least one of the conductors and a plurality of pads provided on a back surface of the imaging element;
a conductor positioning step of positioning the tip ends of the conductors that protrude from the electric wire positioning and fixing body to the pads; and
a conductive material fixing step of blowing high-temperature air having a temperature higher than a melting point of the conductive materials to the conductive materials to conductively connect the pads and the conductors via the melted conductive materials.

10. The cable connection method for the camera module according to claim 9,
wherein in the electric wire positioning and fixing body, the electric wires are inserted into insertion holes of a jig in which a plurality of insertion holes into which the electric wires are inserted are bored such that the electric wires face the pads,
wherein in the electric wire positioning and fixing body, the electric wires before being inserted into the jig are collectively covered by an insulating adhesive material, and
wherein the electric wire positioning and fixing body is formed by removing the jig from the electric wires after the adhesive material is solidified.

11. The cable connection method for the camera module according to claim 9,
wherein the electric wire positioning and fixing body is formed by inserting the electric wires into insertion holes of an insulation member in which a plurality of insertion holes into which the electric wires are inserted are bored such that the electric wires face the pads, and fixing the electric wires and the insulation member by an adhesive material.

12. The cable connection method for the camera module according to claim 9,
wherein the electric wire positioning and fixing body is formed by inserting the electric wires into insertion holes of an electric wire positioning plate in which a plurality of insertion holes into which the electric wires are inserted are bored such that the electric wires face the pads, and adhering the electric wires before being inserted into the electric wire positioning plate to the electric wire positioning plate while collectively covering the electric wires with an insulating adhesive material.
